# EUROPEAN PATENT SPECIFICATION

(11) **EP 1 836 969 B1**
(45) Date of publication and mention of the grant of the patent: **03.06.2009**
(21) Application number: 07104169.3
(22) Date of filing: 14.03.2007
(51) Int. Cl.: A61B 17/00

(54) **Medical apparatus**
Medizinische Vorrichtung
Appareil médical

(30) Priority: 22.03.2006 US 386082
(43) Date of publication of application: 26.09.2007
(73) Proprietor: RADI MEDICAL SYSTEMS AB, 754 50 Uppsala (SE)
(72) Inventor: Preinitz, Fredrik, 753 50, UPPSALA (SE); Egnelöf, Per, Paklok Sub-District, Thalang District Phuket 83110 (TH)
(74) Representative: Holmberg, Nils Anders Patrik

(56) References cited:
- WO-A-20/05092203

## Description

### Field of the invention

The present invention relates generally to a medical device for closing an opening or defect in an organ within a living body, e.g. a septal defect in a heart or a percutaneous puncture in a vessel wall (such as walls in arteries or other blood vessels), and in particular to an expandable and repositionable closure device, which can be remotely maneuvered from an initial positioning configuration to a final configuration in which the opening or defect is closed, and more particularly to a mechanical actuator by which the expandable and repositionable closure device is inserted, positioned and delivered in the opening or defect.

### Background of the invention

The closing of an opening in an organ of a patient is a medical procedure that frequently has to be practiced by doctors or other trained medical personnel. The opening may be a hole created by the doctor for a specific and usually temporary purpose, or the opening can be a congenital or acquired defect. An example of the former would be a puncture hole created in a patient's femoral artery to obtain access to the coronary system, while an example of the latter is a septal defect in a patient's heart. For descriptive and illustrative purposes the present invention will be described with reference to such a septal defect, although such techniques can be applied to other fields of application.

As is well-known, the human heart is divided into four chambers: the left atrium, the right atrium, the left ventricle, and the right ventricle. The atria are separated from each other by the interatrial septum, and the ventricles are separated by the interventricular septum.

Either congenitally or by acquisition, abnormal openings or holes can form between the chambers of the heart, causing shunting of blood through the opening or hole. For example, with an atrial septal defect, blood is shunted from the left atrium to the right atrium, which produces an over-load of the right side of the heart. In addition to left-to-right shunts such as occur in patent ductus arteriosus from the aorta to the pulmonary artery, the left side of the heart has to work harder because some of the blood will recirculate through the lungs instead of going to the rest of the body. The ill effects of such lesions usually cause added strain to the heart with ultimate failure if not corrected.

One way to cure a septal defect in the septum of a heart is to position and anchor a specially designed closure device at the septum such that both sides of the septal defect are spanned by the closure device to thereby close the defect. Examples of such septal defect closure devices are known from the U.S. Patent Nos. 5,853,422; 6,024,756; 6,117,159 and 6,312,446 to Huebsch et al., which disclose a closure device comprising a cylindrical shaft of metal or polymeric material with concentric parallel cuts through the wall of the device to thereby create flattened support struts. The centers of the support struts are intended to move radially away from the longitudinal axis of the device in a hinge-like fashion in response to movements of the proximal and distal ends of the device towards the center thereof. The patents show further a number of different deployment catheters by which the closure device can be positioned and delivered. The deployment catheters are, however, described in a rather rudimentary fashion, and do not seem to include all the members necessary to effect the movements and functions of the closure device.

A similar septal defect closure device is also disclosed in European patent application EP1651116 to Chanduszko.

A further defect closure device is disclosed in WO 2005/092203 including the description of an insertion assembly.

Within the medical field it is of utmost importance that closure apparatuses work properly, and a general object of the present invention is therefore to improve a medical apparatus comprising a closure device of the aforementioned type and an accompanying mechanical actuator in such a way that a safe and user-friendly medical apparatus is obtained, wherein the movements of the closure device can be controlled in a reliable way by the mechanical actuator.

### Summary of the invention

The above-mentioned object is achieved by the present invention according to the independent claim. Preferred embodiments are set forth in the dependent claims.

According to the present invention, a septal defect closure device comprises an elongated essentially tubular member in which a first set of longitudinal slits or cuts have been made on a first side of an uncut central portion and a second set of longitudinal slits or cuts have been made on the opposite side of the central portion. On each side of the central portion, the slits extend towards the ends of the tubular member to terminate a short distance before the respective end, such that uncut proximal and distal end portions are formed. The tubular member, which is made from a flexible and preferably resorbable material, has thereby been provided with proximal and distal sets of struts or ribs. The distal ends of the distal struts are flexibly connected to the distal end portion of the tubular member, while the proximal ends of the distal struts are flexibly connected to the central portion. Similarly, the proximal ends of the proximal struts are flexibly connected to the proximal end portion of the tubular member, while the distal ends of the proximal struts are flexibly connected to the central portion. The struts are further each provided with a hinge section such that each strut in effect is divided into two articulated arms.

When the septal defect closure device during use is compressed such that the distal and proximal end portions are forced towards each other, the hinge sections of the struts move radially out from the longitudinal central axis of the closure device, and the respective arms of the struts assume an essentially perpendicular angle to the central axis of the closure device. The septal defect closure device further comprises a central elongated locking member, which can be either separate from or integrated with the tubular member. In the former case, an elongated locking member is inserted into the tubular member such that the distal end portion of the tubular member abuts one or several radially protruding portions of a distal end of the locking member, and the proximal end portion of the tubular member is then pushed over one or several radially protruding portions of a proximal end of the locking member. The radially protruding portions can comprise a continuous rim or discrete radial protrusions of various dimensions. In the compressed state, the central, proximal and distal portions of the tubular member fit snugly along the central locking member, and the closure device is held in the compressed state by the enlarged distal and proximal rims or other radially protruding portions of the locking member, which prevents the closure device from resuming its original elongated shape. The device further comprises a keying feature, which prevents rotational movement of the locking member in relation to the tubular member.

In accordance with the present invention, the medical closure apparatus comprises further a mechanical actuator by which the closure device can be maneuvered through four (4) well-defined configurations: an introduction configuration, a positioning configuration, a closed configuration, and a locked configuration. The mechanical actuator comprises an actuating member and a holder, whose relative longitudinal translational motion causes the closure device to transform from the introduction configuration via the positioning configuration to the closed configuration, and - if desired - back to the introduction configuration. In a preferred embodiment of the present invention, the actuating member comprises a pusher and a retractor, which move together as a unit to accomplish the first three configurations of the closure device, but which are moved in relation to each other to accomplish the final locked configuration of the closure device. Furthermore, the medical closure apparatus comprises a spring, which has a spring force large enough to move the closure device from the introduction configuration via the positioning configuration to the closed configuration. However, to move the device from the closed to the final irreversibly locked state, force in excess of the spring force is required, which diminishes the risk of prematurely locking the device in place. The medical closure apparatus comprises further a catheter, inside which the mechanical actuator can slide. If the closure apparatus is to be used to close a puncture hole in, for example, a femoral artery, the catheter is preferably replaced with an introducer, which normally has been used during a previous medical procedure and which already is in place in the artery, or is a specially designed introducer.

### Brief Description of the Drawings

Fig. 1 is a schematic illustration of a human heart having an atrial as well as a ventricular septal defect.
Fig. 2 is a schematic illustration of a human heart having a septal defect, which is to be closed by means of a medical procedure that, in a first step, involves the positioning of a septal defect closure device according to the present invention.
Fig. 3 illustrates an intermediate step in the medical procedure, in which a distal portion of the closure device of Fig. 2 is expanded in order to locate the septal defect from the distal side of the septal defect.
Fig. 4 illustrates the closure device of Fig. 2, which has been positioned in the septum to close the septal defect therein.
Fig. 5 shows a septal defect closure device according to the invention in an introduction configuration before any longitudinal compression of the closure device.
Fig. 6 shows the closure device of Fig. 5 in an intermediate semi-compressed positioning configuration.
Fig. 7 shows the closure device of Fig. 5 in another intermediate semi-compressed positioning configuration.
Fig. 8 shows a locking member which according to one embodiment constitutes a separate part of a septal defect closure device.
Fig. 9 shows the closure device of Fig. 5 in a closed configuration.
Fig. 10 shows the closure device of Fig. 5 in another closed configuration, in which distal portions of a mechanical actuator are visible.
Fig. 11 illustrates the closure device of Fig. 5 in a final locked configuration.
Fig. 12 is a sectional drawing of a medical apparatus according to the present invention shown in an introduction stage of a closure delivering operation.
Fig. 13 is a sectional drawing of the medical apparatus of Fig. 12 shown in a positioning stage of the closure delivering operation.
Fig. 14 is a sectional drawing of the medical apparatus of Fig. 12 shown in a closing stage of the closure delivering operation.
Fig. 15 is a sectional drawing of the medical apparatus of Fig. 12 shown in a locking stage of the closure delivering operation.
Fig. 16 is a sectional drawing of the medical apparatus of Fig. 12 shown in a releasing stage of the closure delivering operation.

### Detailed Description of Preferred Embodiments of the Invention

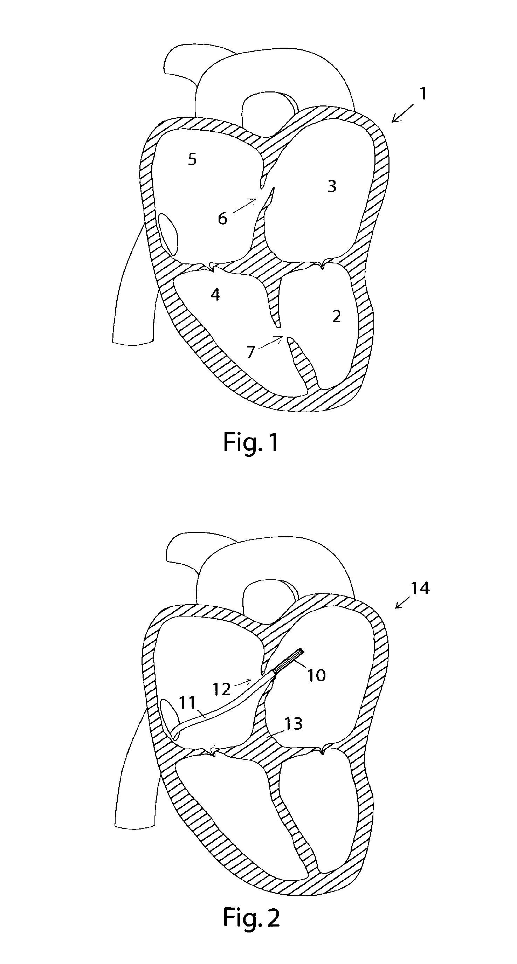

A schematic cross-sectional view of a human heart 1 is shown in Fig. 1. The heart 1, with its left ventricle 2, left atrium 3, right ventricle 4, and right atrium 5, suffers from an atrial septal defect 6 as well as a ventricular septal defect 7. Below a medical procedure will be discussed in which an atrial septal defect is closed. It should, however, be clear that a septal defect closure device according to the present invention equally well could be employed to close a ventricular septal defect such as ventricular septal defect 7 of Fig. 1. It should further be noticed that the septal defects 6, 7 can be accessed from different vessels, e.g. from the superior or inferior vena cava, or from the aorta. This implies, in turn, that throughout the present description terms like "distal" and "proximal" should always be seen from the end of a delivering catheter, through which a septal defect closure device is delivered (and not from any particular chamber or vessel of a heart).

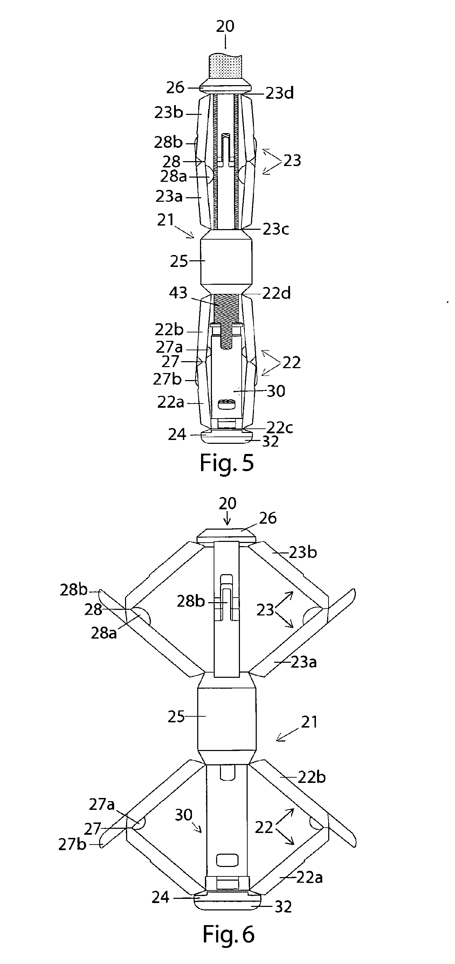

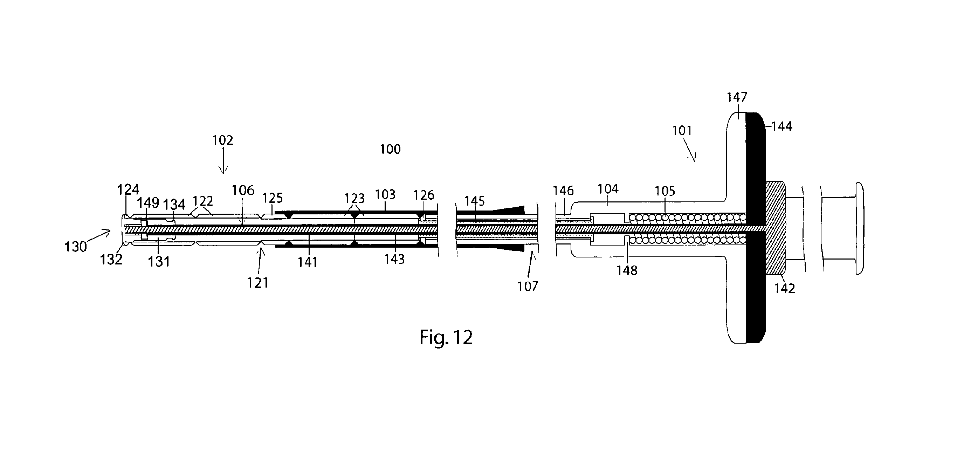

In conjunction with Figs. 2 to 4, a medical procedure will be briefly described, in which a medical apparatus comprising a septal defect closure device is employed to close a septal defect in the septum of a heart; and thereafter different parts and functions of the closure device itself will be described in detail in conjunction with Figs. 5 to 11. The medical apparatus comprises further a mechanical actuator whose operation is described with respect to Figs. 12 to 16.

Fig. 2 illustrates a septal defect closure device 10 which by use of a mechanical actuator (not shown in Fig. 2) has been introduced into an atrial septal defect 12 in the atrial septum 13 of a heart 14 using a delivering catheter 11. The closure device 10 is of the same general construction that has been generally described above, and comprises an elongated tubular member in which distal and proximal sets of struts have been provided. The distal struts extend from a central portion of the closure device 10 to a distal end portion thereof, and the proximal struts extend from a proximal end portion of the closure device 10 to the central portion. As already discussed, each strut is provided with a hinge region, and each strut is thereby effectively divided into two hinge-connected arms. The characterizing details of the hinge region will be discussed further below. In Fig. 2, the closure device 10 is shown in an initial introduction configuration, in which the arms of each strut are substantially aligned with each other. In this introduction configuration, the closure device 10 therefore has a generally elongated tubular shape, which facilitates the introduction of the closure device 10 into the artery and heart of a patient. The introduction configuration is defined as the configuration that the closure device assumes by itself, i.e. without any compression being induced by a mechanical actuator (not shown in Fig. 2) connected to the closure device. In this introduction configuration, the closure device has therefore a generally tubular shape, although the closure device could be preformed such that the arms of each strut exhibit a small positive angle in relation to each other. Such a positive angle guarantees the proper radial expansion of the tubular member during longitudinal compression of the tubular member.

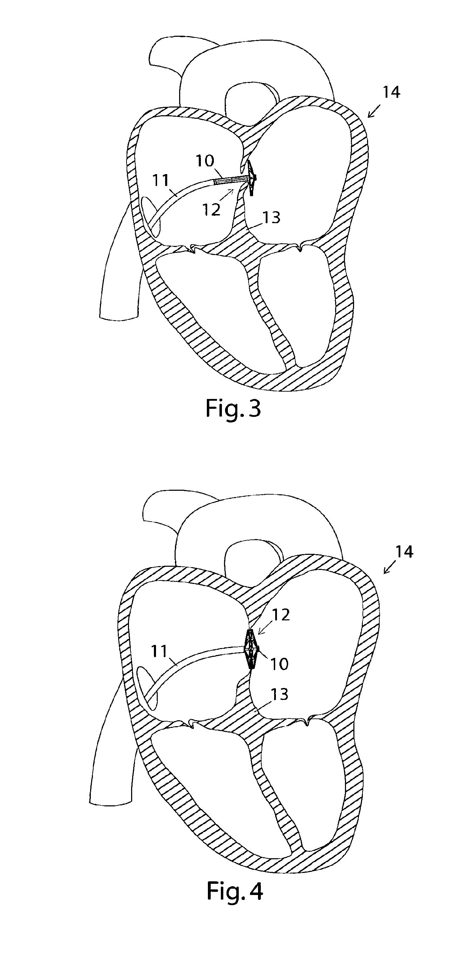

To ascertain correct positioning of the closure device 10 with respect to the septal defect 12, the distal set of struts can be moved radially outwards from the central axis of the closure device 10, such that a partly expanded configuration is obtained. The radial movements of the distal struts are effectuated by partially compressing the closure device 10 through the maneuvering of a mechanical actuator (not shown in Figs. 2-4). In this semi-expanded locating or positioning configuration, the closure device 10 is retracted until the distal struts abut the distal side of the atrial septum 13 surrounding the septal defect 12. The septal defect 12 can thereby be located by a doctor, who in this phase of the medical procedure will feel a marked increase in resistance against further retraction. This intermediate step of the medical procedure is depicted in Fig. 3. The function and operation of the mechanical actuator that effectuates the semi-expanded configuration shown in Fig. 3 are thoroughly explained below. It should be noted that it is within the scope of the invention that a similar locating procedure can be performed by compressing the device such that the proximal struts are expanded, and thereafter advancing the closure device until the proximal struts abut the proximal side of the atrial septum (not shown in the figures), or alternatively by expanding both the distal and the proximal struts.

When the atrial septum 13 and thereby the septal defect 12 have been correctly located, the closure device 10 is fully expanded such that the proximal struts as well as the distal struts are forced radially outwards by maneuvering of the mechanical actuator mentioned above. In this septal defect closing configuration, the closure device 10 spans both the distal side and the proximal side of the septal defect 12, and is then held in this position. As can be seen in Fig. 4, the closure device 10 sandwiches the atrial septum 13 to thereby close the septal defect 12 therein. It should be mentioned that the term "close" or similar terms used herein in conjunction with the description of the closing of a septal defect should not be taken too literally. Such terms are meant to encompass all stages from actually sealing or closing off a septal defect to merely restricting the flow of blood therethrough, the important feature being that the closure device permits and facilitates healing of the septal (or other type of) defect over time. To improve the sealing capability of a closure device of the present type, it is conceivable that the distal and/or proximal struts at least partly are covered by a thin membrane or formed integrally with a thin membrane, which preferably is made from a resorbable material.

A special feature of the closed configuration illustrated in Fig. 4 is that the closure device 10 still is repositionable. This means that by using a mechanical actuator (not shown in Fig. 4), the closure device 10 is reversibly movable between the configurations described above in conjunction with Figs. 2-4, i.e. from the closed configuration of Fig. 4, to the intermediate positioning configuration of Fig. 3, and back to the original introduction configuration of Fig. 2. The closure device 10 can then be retracted out of the patient's body and be disposed, or can once again be positioned by repeating the steps illustrated above. The closed configuration of the closure device 10 is defined as the extreme end position of the different and gradually changing positioning configurations. In the closed configuration essentially no further compression of the closure device 10 is possible while still maintaining a reversibly movable closure device 10. The latter will be thoroughly discussed below.

In accordance with the present invention, a closure device encompasses a fourth configuration, in which the closure device is irreversibly locked. The transition from the closed configuration to this locked configuration is effectuated by the mechanical actuator mentioned above. A special feature of the present closure device is that a doctor will feel when the closed configuration has been reached, so that he or she can decide whether the mechanical actuator should be maneuvered such that the final locked configuration is achieved. Having in mind that the closed configuration constitutes a situation from which the closure device can be removed, whereas the locked configuration implies a non-retrievable closure device, the importance of having a well-defined transition between these two states should be appreciated. Also this feature and how the locked configuration is achieved by a mechanical actuator will be further discussed below.

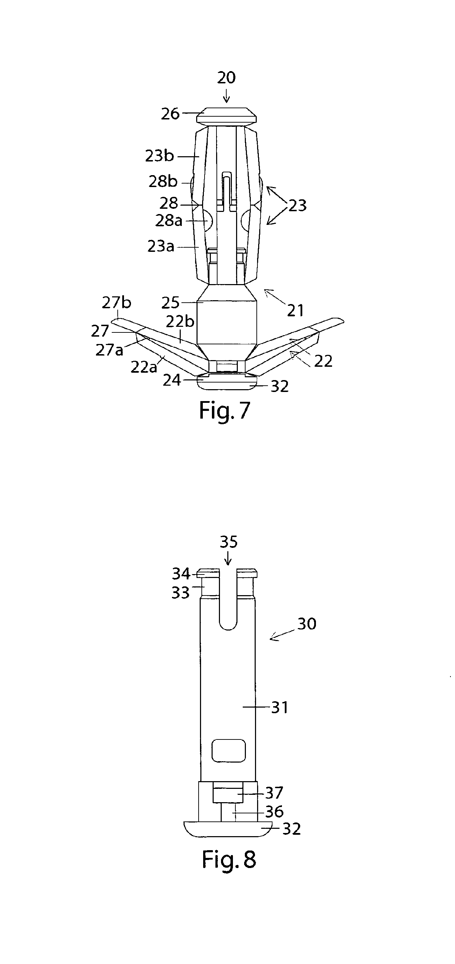

An embodiment of a septal defect closure device 20 according to the present invention is illustrated in Fig. 5. Fig. 5 shows the closure device 20 in a first or introduction configuration in which the closure device 20 has the general shape of an elongated tubular member 21, through which a number of longitudinal, parallel cuts or slits have been made to thereby form a first or distal set of struts 22 and a second or proximal set of struts 23. The first strut set 22 extends between a first end portion 24 of the tubular member 21 and a central portion 25 thereof, while the second strut set 23 extends between the central portion 25 and a second end portion 26 of the tubular member 21. The first and second end portions 24, 26 as well as the central portion 25 are uncut and are preferably shorter than the slit portions of the tubular member 21. Somewhere along the length of the first set of struts 22, the tubular member 21 has been provided with a hinge region 27, which will thereby act as a hinge or articulation 27, effectively dividing each strut 22 into two articulated arms: a first or distal arm 22a and a second or proximal arm 22b. Similarly, the struts in the second set of struts 23 are each provided with a hinge section 28, which in effect divides each strut 23 into two articulated arms: a first or distal arm 23a and a second or proximal arm 23b. In Fig. 5, the device has been provided with two distal arms 22 and four proximal arms 23; however the number of arms for each set can be varied without departing from the scope of the present invention, as will be further described below. In addition, the closure device 20 comprises a locking member 30, which is further described below in connection with Fig. 8.

In the figures, the hinge region has been constructed so as to comprise a middle longitudinally extended section which will constitute an extra protruding arm 27b, 28b when folded, which is more apparent in Figs. 6-7. In addition, the hinge regions have been provided with a protrusion 27a, 28a, which serves to keep the two connected arms 22a, 22b or 23a, 23b at an angle towards each other even in the initial introduction configuration of the tubular member, as described above. This protrusion encounters either a central holder member 43 or a locking member 30, if present, in the initial configuration of the arms, which prevents the two connected arms from aligning completely with each other. It should however be noted that it is within the scope of the present invention that the central hinge regions can be constructed in a less complex manner, e.g. by merely removing material on the outer side of the struts, thus creating weakened sections that will act as hinges, lacking the protruding extra arms 27b, 28b and/or the protrusions 27a, 28a on the inside of the hinge region.

It is should further be emphasized that the term "tubular" is merely intended to indicate the general shape of an elongated, hollow member, which comprises a number of struts, the ends of which are connected to ring-shaped or essentially cylindrical members, and which in a first introduction configuration assumes an essentially tubular shape. In other words, a tubular member, like tubular member 21, does not actually have to be cut or slit in order to create distal and proximal struts. On the contrary, a tubular member, having struts with hinge regions, as well as ring-shaped or essentially cylindrical central, distal and proximal end portions, can advantageously be directly produced in this form, e.g. by injection molding or die casting. Furthermore, the struts of a tubular member, like tubular member 21, do not have to be exactly aligned with each other. Instead, a tubular member can be preformed in such a way that the two arms of a strut exhibit an angled relation to each other, to thereby guarantee that the arms actually bend outwards during compression of the tubular member. Nevertheless, the definition of the introduction configuration is still the configuration or state wherein a closure device has not been subjected to any compression by means of a mechanical actuator. The introduction configuration may therefore also be regarded as the "natural" state of the closure device.

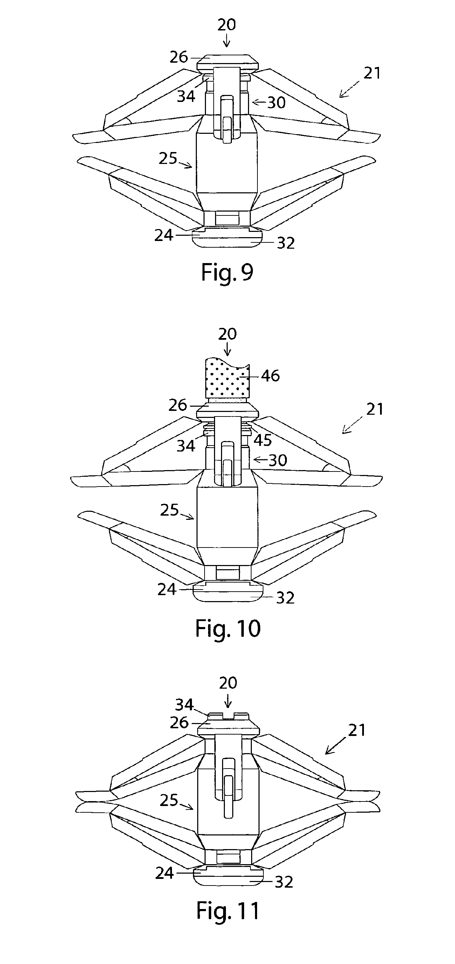

In Fig. 6, the closure device 20 of Fig. 5 is depicted in a semi-expanded positioning configuration in which the distal and proximal end portions 24, 26 of the closure device 20 have been moved towards the central portion 25. The hinge sections 27, 28 of the first and second struts 22, 23 have thereby been forced to move outwards from the central axis of the closure device 20, and the articulated arms 22a, 22b and 23a, 23b have assumed a more angled relation to the central axis of the closure device 20. The semi-expanded configuration shown in Fig. 6 can be used to determine the proper position for the closure device 20, and can also be regarded as a positioning configuration prior to a closed configuration described below in conjunction with Fig. 9 or Fig. 10. The positioning configuration is defined as all intermediate states between the introduction configuration defined above and the closed configuration, which will be further described and defined below. Another example of a positioning configuration is illustrated in Fig. 7.

As can be seen in Figs. 5-7, the closure device 20 comprises further a locking member 30, which is separately illustrated in Fig. 8. The locking member 30, which according to the invention can constitute either a separate or an integrated part of closure device 20, or may be an integrated part thereof, comprises a hollow tubular body 31, which along the central part of its length is provided with an outer diameter approximately equivalent to the inner diameter of the tubular member 21 of the closure device 20. More specifically, the locking member 30 comprises a distal end rim 32, an intermediate body 31, a proximal groove 33, and a proximal end rim 34. The distance between the distal end rim 32 and the proximal end rim 34 is considerably smaller than the length of the tubular member 21. As the observant reader already may have appreciated, the diameter of the distal end rim 32 is larger than the inner diameter of the distal end portion 24 of the tubular member 21. In turn, the inner diameter of the distal end portion 24 is marginally larger than the diameter of the intermediate portion 31 of the locking member 30, such that the distal end portion 24 of the tubular member 21 can slide over the locking member 30 until the distal end portion 24 abuts the distal end rim 32 In this position, as seen in e.g. Figs. 5-6, locking knob 37 functions by locking the distal end portion 24 against the distal end rim 32, such that the tubular member and the locking member are locked at their distal ends. In addition, to prevent rotational movement of the tubular member 21 relative to the locking member 30, a keying feature between the locking member and the tubular member is provided. In one embodiment, shown in part in Fig. 8, the locking member is supplied with a protrusion 36, which is matched by a recess in the inner surface of distal end portion 24 (not shown in the figures).

Similarly to the inner diameter of distal end portion 24, the inner diameters of the central portion 25 and the proximal end portion 26 of the tubular member 21 are marginally larger than the outer diameter of the intermediate portion 31 of the locking member 30. Additionally, the outer diameter of the proximal end rim 34 is slightly larger than the inner diameter of the proximal end portion 26. During use, the proximal end portion 26 of the tubular member 21, which is made from a somewhat elastic material, must therefore be forced over the proximal end rim 34 and can then slide on the intermediate portion 31. As can be seen in Fig. 8, the locking member 30 preferably comprises a recess 35, which in combination with the groove 33 provide the proximal end rim 34 with certain resilience, facilitating the sliding of the proximal end portion 26 of the closure device 20 over the proximal end rim 34 of the locking member 30.

In the embodiment of the locking member shown in Fig. 8 the distal and proximal ends 32, 34 comprise a completely circumferential circular rim and a discontinuous circumferential rim, respectively. It should be noted that the locking member's distal and proximal ends can comprise other shapes, provided that the distal end 32 supplies a point of hindrance for the distal end portion 24 and that the proximal end 34 both allows proximal end portion 26 to be forced over it and locks the final expanded state of the device in place. Many shapes are conceivable such that at least one outer cross-sectional dimension of the distal end 32 is larger than at least one inner cross-sectional dimension of the end portion 24 and at least one outer cross-sectional dimension of the proximal end 34 is adapted to, i.e. is slightly larger than, at least one inner cross-sectional dimension of the end portion 26. One such possibility is to supply the ends with one or several radial protrusions.

As indicated above, the closure device 20 can assume an infinite number of positioning configurations during a positioning operation in which a septal defect is located and the closure device 20 is positioned therein. According to the present invention, there is, however, a well-defined endpoint for the positioning operation. This endpoint, which is referred to as the closed configuration of the closure device 20, is illustrated in Fig. 9, where it can be seen that the central portion 25 of the tubular member 21 has been positioned over the intermediate portion 31 of the locking member 30, while the proximal end portion 26 of the tubular member 21 abuts the proximal end rim 34 of the locking member 30. As has been mentioned above, the inner diameter of the proximal end portion 26 is slightly less than the diameter of the proximal end rim 34, which implies that further compression of the tubular member 21 is not possible - unless extra force is applied such that the proximal end portion 26 is forced over the proximal end rim 34. The closed configuration of Fig. 9 thereby constitutes a well-defined state.

However, the definition above, that a closed configuration is a configuration in which no further compression of the tubular member 21 is possible without forcing proximal end portion 26 over proximal end rim 34 allows for additional examples of a closed configuration. In practice, the movements of the closure device are effectuated by the previously mentioned mechanical actuator, parts of an example of which are illustrated in Fig. 10 together with the tubular member 21 as well as the locking member 30. The mechanical actuator comprises a holder and an actuating member. The holder comprises a holder member 43 (not seen in Fig. 10, refer to Fig. 5) and a locking pin (not seen in Fig. 10), while the actuating member comprises a pusher 46 and a retractor 45. By moving the actuating member back and forth, a doctor can during a preceding positioning operation let the tubular member 21 assume different positioning configurations, to thereby locate a septal defect (or some other type of tissue opening, e.g. a percutaneous puncture in an artery wall) and position the closure device 20 in the opening of the defect. The terms "actuating member" and "holder member" should not be interpreted too literally; it is actually the relative motion between these two members that is important. In other words, seen from some fixed position it could actually be the holder that is moved, whereas the actuating member is still. Further, during the first three stages, i.e. the introduction, positioning and closed configuration of the closure device 20, the pusher 46 and the retractor 45 move together as a first unit, while the holder member 43 and the locking pin move together as a second unit. In the situation illustrated in Fig. 10, the distal end of the retractor 45 abuts the proximal end rim 34 of the locking member 30. Thus, Fig. 10 illustrates a well-defined end position for the positioning operation, in which no further compression of the tubular member 21 is possible by maneuvering of the actuating member in relation to the holder without forcing proximal end portion 26 over proximal end rim 34. If, on the other hand, an actuating member were engaged inside a proximal end portion of a tubular member, the situation would resemble the situation illustrated in Fig. 9, i.e. a well-defined end point of the positioning operation - in which no further compression of the tubular member is possible without forcing an end portion over an end rim - would be when a proximal end portion of the tubular member abuts a proximal end rim of a locking member. The closed configuration of a closure device according to the present invention is thereby defined as the extreme end position of the positioning configurations, wherein an end portion of a locking member prevents further compression of a tubular member. This definition also encompasses closure devices where a proximal end portion of a locking member prevents further compression of a tubular member, i.e. a closure device where a distal end portion of a tubular member is pulled over an enlarged distal end rim of a locking member rather than - as in the closure device described above - having a proximal end portion of a tubular member that is pushed over an enlarged proximal end rim of a locking member. It should be kept in mind that, as mentioned above, the locking member's distal and proximal ends can also comprise other shapes, such as any variation of radial protrusion, as long as the cross-sectional dimensions of the end portions allow the progression through the four different configurations as described herein.

From Fig. 10 it may be realized that when the retractor 45 abuts the proximal end rim 34 of the locking member 30, the closure device 20 can be transferred into the final locked state by movement of the pusher 46. To accomplish this, the pusher 46 (which can slide with respect to retractor 45) is advanced, so that the proximal end portion 26 of the tubular member 21 is forced up and over the proximal end rim 34 of the locking member 30. This movement requires that the proximal end portion 26 and/or the proximal end rim 34 possesses a certain degree of resilience.

The final locked configuration of the closure device 20 is illustrated in Fig. 11, in which the distal and proximal end portions 24, 26 of the tubular member 21 have been fully moved towards each other until the central portion 25 is positioned over the tubular body 31 of the locking member 30 and the proximal end portion 26 has been moved over the proximal end rim 34 of the locking member 30. The closure device 20 is held in this compressed state due to the enlarged distal and proximal end rims 32, 34 of the locking member 30, which have diameters larger than the distal end portion 24 and the proximal end portion 26, respectively. The closure device 20 can then be released and left in this locked configuration by maneuvering of the holder member and locking pin mentioned above. The locked configuration of a closure device is thereby defined as the configuration in which the closure device is fully expanded, and in which the closure device can be held without assistance of a mechanical actuator.

It should be noted that it is also within the scope of the invention to vary specific dimensions of the closure device, which provides the advantage of being able to adapt a closure device to both the particular anatomy of the underlying tissue and the local physiology (e.g. the flow or coagulation tendency of the blood). It may, for example, be desirable to arrange a closure device in such a way that the left part of the closure device, i.e. the part that is implanted into the left atrium of a heart, is smaller than the right part of the closure device, to thereby reduce the amount of artificial material introduced into the left atrium, which in turn may reduce the formation of thrombogenic material therein. One example of varying the device's dimensions is to provide a closure device having proximal struts with one length and distal struts with a different length. Another is to vary the length and/or outer diameter of a central portion of a tubular member, thereby creating the possibility to adapt the device to a particular anatomy of the underlying tissue. Similarly, it is also possible to have different lengths of the articulated arms within a strut set, such that, for example, the distal arms are longer than the proximal arms, or vice versa. Furthermore, it is also within the scope of the invention to use any number of struts per set, preferably between 1 and 10 struts per set, more preferably between 2 and 6 struts per set. In addition, it can be advantageous to provide a closure device in the form of two separate tubular members (and a separate locking member) as this would provide a doctor with the possibility to tailor a septal defect closure device to the specific medical situation at hand, without the necessity of producing an excessive large number of closure devices with different dimensions.

In this context, it should be recognized that it is not mandatory that a heart is accessed via the venous system, as is shown in Figs. 2 to 4, but the heart could be accessed via the arterial side. This implies that if a doctor wishes to place a smaller part of a closure device at the left side of a heart than at the right side of the heart, then this smaller part (e.g. the shorter struts) will constitute the distal set of struts if the heart is accessed via the venous system, whereas the smaller part will constitute the proximal set of struts if the heart is accessed through the arterial system.

It has already been mentioned that a locking member can constitute a separate part of a closure device, and a locking member can be made from a first material and a tubular member can be made from a second material. With different materials some specific advantages can be achieved. If, for example, the closure device is a resorbable closure device, then the resorption time of the material in the locking member can be different from the resorption time of the material in the tubular member, such that the mechanical properties of the closure device are maintained until the surrounding tissue has healed to the point where the support of the closure device is not necessary anymore. Further, whether or not the materials are resorbable materials, different requirements are put on the different pieces. For example, the material in the hinge portions of a tubular member must be ductile and have a high durability, whereas the locking member must have a rather high stiffness. Furthermore, it may be necessary to have one material in a locking member and another material in a tubular member in order to match the resorption times due to different dimensions of the members involved in a resorbable closure device.

Examples of resorbable materials for the tubular member and the locking member may include, but are not limited to, those materials made from aliphatic polyesters, polyether esters, and polycarbonates. More specifically, synthetic resorbable polymers such as homopolymers and copolymers made from any of the monomers lactide, glycolide, epsilon-caprolactone, trimethylene carbonate, and paradioxanone are advantageous because of their long clinical use. Other lactones that may be used together with any of the abovementioned monomers to make copolymers of various properties are valerolactone, b-butyrolactone and dioxepanone, however also other 4, 5, 6 and 7 member lactones may be of interest to obtain the characteristic material properties needed to fulfill a smooth operation of the invented closure device.

The tubular member could preferably be made from a semi-crystalline material with a lower modulus than the locking member. As previously stated, the device could, e.g. because of the hinge portions, have a more flexible material in the tubular member. Such material is preferably made from a block copolymer characterized by having a soft middle part distinguished by having a glass transition temperature below body temperature and a semi-crystalline part at each end of the soft middle part. The semi-crystalline part could be polymerized from any of the monomers glycolide, lactide, or paradioxanone. Since polyparadioxanone is a relatively soft and pliable material compared to polyglycolide and polylactide, the tubular member can be made from pure polyparadioxanone itself.

The locking member can be made from any of the above materials, but to secure the locking mechanism it is advantageous if the material is stiffer than the material used in the tubular member. The material should also preferably resorb at a somewhat slower pace than the tubular member. The locking member could also be made from amorphous or semi-crystalline material, and preferably from homopolymers or copolymers where the main monomer component is lactide, caprolactone, or paradioxanone.

Further examples of synthetic resorbable polymers that may be used in the tubular or locking member of the invented closure device are resorbable polymers made from dicarboxylic acids such as succinic, glutaric, adipic or pimelic acids with various forms of diols, polymers composed of segmented blocks of polyethyleneglycol and butyleneterephthalate, various forms of tyrosine carbonate polymers, phosphazene polymers, orthoester polymers or resorbable polyurethanes.

A particular advantage of the groups of synthetic resorbable polymers mentioned above is that various mechanical properties can be accomplished by simply changing the monomer composition in the homopolymer or copolymer. Further, in contrast to natural biopolymers, these materials can be molded and machined into complex structures, and by varying the monomer composition large time spans can be achieved for their resorption times.

It may be appreciated that it can be advantageous to provide a radiopaque closure device which is visible in an X-ray machine. When the closure device is made from a synthetic resorbable polymer, a radiopaque closure device can conveniently be produced by mixing the polymer with a suitable radiopaque agent. A suitable radiopaque agent is barium sulfate, which can be blended into the polymer or copolymer in an amount between 5% and 50%, and more preferably in an amount of 15% to 30%, to obtain the opacity needed in order to locate the closure device during an X-ray observation. Radiopaque materials can be used in a tubular member of the closure device, but is preferably used in the locking member, which marks the center of the device. The radiopaque agent, e.g. barium sulfate, can be - instead of being mixed with the polymer - introduced into preformed holes in the closure device, which are then sealed by a synthetic resorbable material. As an alternative, preformed holes can be plugged with a resorbable material containing a large amount of a radiopaque agent, e.g. barium sulfate.

Fig. 12 illustrates a medical apparatus 100 comprising a mechanical insertion and delivery assembly 101, a closure device 102, and a delivering catheter 103. The closure device 102 is of the same general design that has been described above, and comprises an elongated tubular member 121 and a locking member 130. The tubular member 121 comprises a first or distal set of struts 122, which extend from a first or distal portion 124 of the tubular member 121 to a central portion 125 thereof. The tubular member 121 comprises further a second or proximal set of struts 123, which extend from the central portion 125 to a second or proximal portion 126 of the tubular member 121. The locking member 130 comprises a hollow tubular body 131, a distal end rim 132, and a proximal end rim 134. The body 131 of the locking member 130 is generally hollow, except for its most distal portion which is solid to avoid leakage of blood through the locking member 130. The insertion assembly 101 comprises a housing 104, a coil spring 105, a holder 106, and an actuator 107. The holder 106 is comprised of a locking pin 141, a locking pin handle 142, a tubular holder member 143, and a holder handle 144. The actuator 107 comprises a tubular retractor 145, a tubular pusher 146, and an actuator handle 147. Both the pusher 146 and the actuator handle 147 are integrated parts of the housing 104, while the retractor 145 is slidably arranged inside the tubular pusher 146. The coil spring 105 is mounted in the housing 104, with a distal end of the coil spring 105 being supported by an internal support member 148 in the housing 104 and a proximal end of the coil spring 105 being supported by the holder handle 144, such that the coil spring 105 strives to increase the distance between the holder handle 144 and the actuator handle 147. The distal end of the tubular holder member 143 is cut and bent outwards into two grip members 149, which are engaged in a recess or cavity in the interior of the hollow body 131 of the locking member 130. The grip members 149 are prevented from approaching each other, i.e. being squeezed together, by the locking pin 141, which in this stage of a delivering operation is disposed within the tubular holder member 143. The distal end of the tubular retractor 145 is upended; and the proximal portion 126 of the tubular member 121 is fixedly arranged between the upended distal end of the retractor 145 and the distal end of the pusher 146. The distal portion 124 of the tubular member 121 has been threaded onto the hollow body 131 of the locking member 130, and abuts the distal end rim 132 of the locking member 130. As will be further elucidated below, except for in the penultimate stage of a delivering operation, the retractor 145 and the pusher 146 move together as a unit in response to reciprocal movements of the actuator handle 147 in relation to the holder handle 144. Since the tubular member 121 is fixated at the actuator 107 at one end and at the distal portion of the locking member 130 at the other end, the corresponding relative motion between the holder 106 and the actuator 107 causes the tubular member 121 to compress or expand longitudinally.

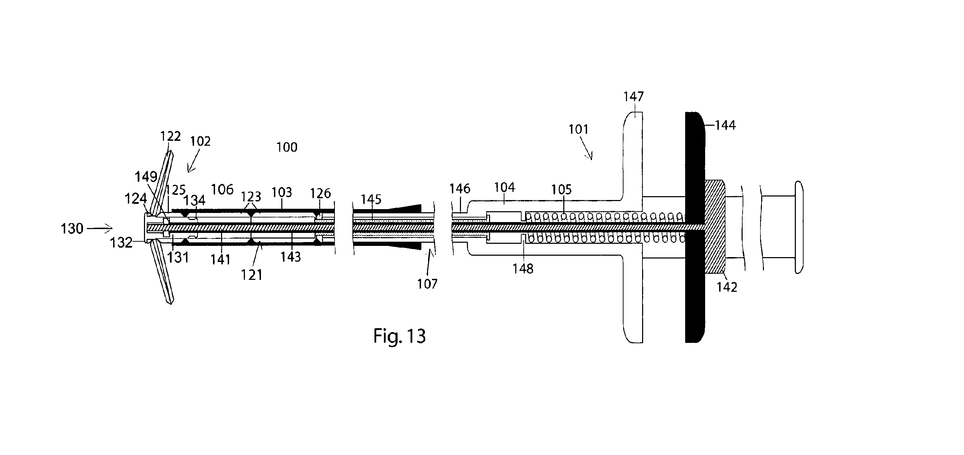

In Fig. 12, the medical apparatus 100 is shown in an introduction phase, in which a distal portion of the tubular member 121 has been advanced out of the delivering catheter 103, whereas the more proximal portion of the tubular member 121 still is within the delivering catheter 103. It can further be noted that the holder handle 144 and the actuator handle 147 abut each other, which corresponds to maximal compression of the coil spring 105. To proceed from the introduction configuration of Fig. 12 to a positioning configuration as described above, the actuator handle 147 is advanced in relation to the holder handle 144, which means that the actuator 107 moves relative to the holder 106. More specifically, this relative movement causes the distal ends of the retractor 145 and pusher 146 to come closer to the distal end of the holder member 143; and, because the tubular member 121 is fixated between the holder 106 and the actuating member 107, the distal struts 122 of the tubular member 121 move outwards and assume an essentially perpendicular angle to the longitudinal central axis of the closure device 102, as has been described above. This stage of a delivering operation is illustrated in Fig. 13.

Fig. 13 shows the medical apparatus 100 in a positioning configuration, in which the distal struts 122 of the tubular member 121 have been expanded in response to a relative motion between the holder 106 and the actuator 107. The positioning configuration of Fig. 13 has been effectuated by the coil spring 105 in that when the distal struts 122 of the tubular member 121 are advanced out of the delivering catheter 103, the action of the coil spring 105 separates the holder handle 144 from the actuator handle 147, which reduces the distance between the grip members 149 at the distal end of the holder member 143 and the proximal portion 126 (held between the upended distal end of the retractor 145 and the pusher 146), and causes the distal struts 122 to assume an essentially perpendicular angle to the central axis of the closure device 102. It should be noted here that the terms "holder" and "actuator", and terms that are derived therefrom, e.g. "holder member", "holder handle" and "actuator handle", should not be given any particular inherent meaning as far as it comes to their corresponding movements. It is the relative motion between the holder 106 and the actuator 107, and - to be described below - between other parts, e.g. retractor 145 and pusher 146, of the insertion assembly 101 that accomplishes the desired effect on the closure device 102.

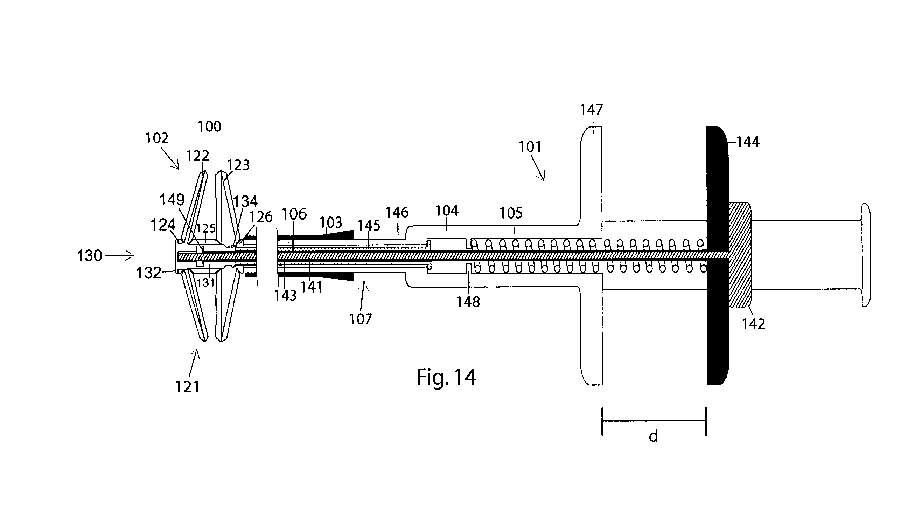

As already has been discussed in conjunction with Fig. 3, the positioning configuration illustrated in Fig. 13 is used to locate a tissue wall, e.g. a septum in a heart, or a vessel wall, and to position the distal struts 122 of the tubular member 121 in contact with the distal surface of this tissue wall. This is accomplished by retracting the insertion assembly 101 until the distal struts 122 abut the distal surface of the tissue wall. The delivering catheter 103 is subsequently retracted relative to the insertion assembly 101 such that also the proximal struts 123 of the tubular member 121 are outside the distal end of the delivering catheter 103; and the closure device 102 is compressed such that the tissue wall is sandwiched between the distal struts 122 and the proximal struts 123 of the tubular member 121. The closed configuration of the closure device 102 is depicted in Fig. 14, which shows that the distance between the holder handle 144 and the actuator handle 147 has been further increased, which, in turn, means that the distance between the distal end of the holder member 143 and the distal end of the pusher 146 has been reduced and caused both the distal struts 122 and proximal struts 123 to assume an essentially perpendicular angle to the central axis of the closure device 102. It should be noted that also this movement is accomplished by the force exerted by the coil spring 105. In Fig. 14 it should in particular be noted that the closure device 102 still is fixedly arranged between the holder 106 and the actuator 107, which means that the whole procedure can be reversed. In other words, by pressing together the holder handle 144 and the actuator handle 147, the closure device 102 can again be transformed to the delivering configuration illustrated in Fig. 12, and the whole medical apparatus 100 could be retracted out of a patient's body and be disposed, or the steps shown in Figs. 12, 13 and 14 could be repeated in order to reposition the closure device 102 in an opening or defect in an organ.

If a doctor determines that the closure device 102 is correctly positioned in an opening or defect in an organ, and, consequently, that the delivering operation described in conjunction with Figs. 12, 13 and 14 has been successful, he or she may decide to irreversibly leave the closure device 102 in the organ, to permanently close the opening or defect therein. To this effect, the closure device 102 has to be transferred from the closed configuration of Fig. 14 to a locked configuration. Here it should be noted that in Fig. 14 the distal end of the retractor 145 abuts the proximal end rim 134 of the locking member 130; and, since the inner diameter of the tubular retractor 145 is smaller than the diameter of the proximal end rim 134 of the locking member 130, the retractor 145 cannot be advanced any further in relation to the grip members 149 at the distal end of the holder member 143.

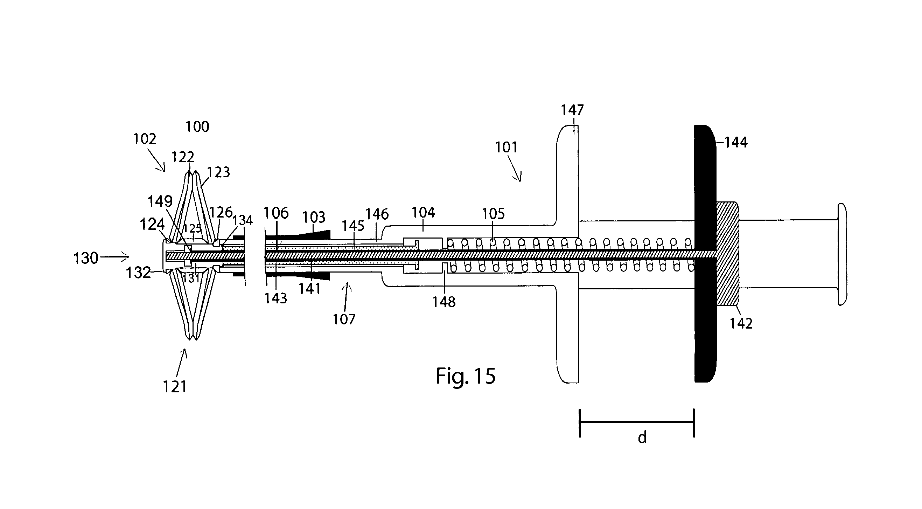

Fig. 15 illustrates the medical apparatus 100 in a configuration in which the closure device 102 is irreversibly locked. By comparing Fig. 14 and Fig. 15 it can be noted that the distance (d) between the holder handle 144 and the actuator handle 147 has been slightly increased in the locked configuration of Fig. 15 compared to the closed configuration of Fig. 14, which means that the pusher 146 has been moved closer to the distal end of the holder member 143. As was explained above, the retractor 145, which is slidably arranged in the tubular pusher 146, can, however, not be moved any closer to the distal end of the holder member 143, resulting in the pusher 146 pushing the proximal portion 126 of the tubular member 121 over the proximal end rim 134 of the locking member 130. In this stage of a delivering operation, the retractor 145 and the pusher 146 no longer move in common as a unit, instead it is the relative motion between the pusher 146 and the retractor 145 that accomplishes the transition from the closed configuration of Fig. 14 to the locked configuration shown in Fig. 15.

It should be emphasized that a unique feature of the present invention is that the spring force exerted by the coil spring 105 is large enough to transfer the closure device from the initial elongated configuration (Fig. 12), via the positioning configuration (Fig. 13) and all the way through to the closed configuration (Fig. 14), but is nonetheless too small to cause the retractor 145 to move relative to the pusher 146. The required force depends on the frictional forces present in the closure device. In light of this requirement, the spring force exerted by the coil spring 105 is preferably within the range of 0.05 N and 20 N. As the locked configuration can not be achieved solely by the coil spring, it is instead achieved by a user who manually advances the actuator handle 147 relative to the holder handle 144. It may be appreciated that this feature is particularly advantageous, because - as has been emphasized before - the closed configuration represents a reversible state from which the closure device 102 can be reversibly operated by the mechanical insertion and delivery assembly 101, whereas the locked configuration is a state from which the closure device 102 cannot be retrieved from a patient's body, at least not without an extensive medical intervention, and the possibility of an unintentional locking of the closure device 102 should therefore be avoided.

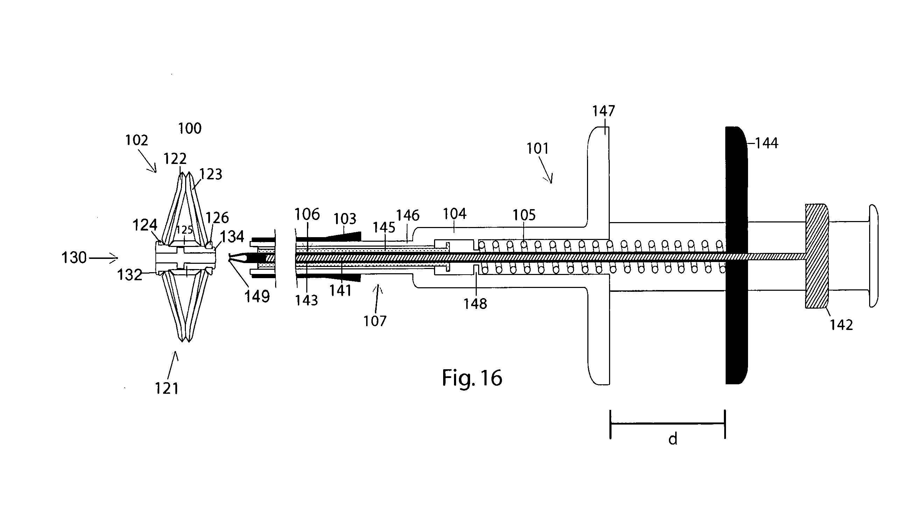

The last important operation that a doctor carries out is to release the closure device 102 from the insertion assembly or tool 101. This is done by retracting the locking pin handle 142 in relation to the holder handle 144, i.e. the locking pin 141 is moved relative to the holder member 143. More specifically, by retracting the locking pin handle 142 such that the locking pin 141 is withdrawn proximally of the grip members 149 at the distal end of the holder member 143, the grip members 149 are free to approach each other; and, in response to a retracting movement of the mechanical insertion tool 101, the grip members 149 are disengaged from the recess or cavity in the interior of the hollow body 131 of the locking member 130. Fig. 16 illustrates how the closure device 102 is released from the mechanical insertion assembly 101, and illustrates further that the grip members 149 were preformed with a tendency to point towards each other. Optionally, an insertion assembly, like insertion assembly 101, may be provided with a safety mechanism that prevents the withdrawal of a locking pin before an actuator handle has been moved to its outmost position with respect to a holder handle, to avoid the risk of unintentionally disengaging a closure device from an insertion assembly.

Although the present invention has been described with reference to specific embodiments, also shown in the appended drawings, it will be apparent for those skilled in the art that many variations and modifications can be done within the scope of the invention as described in the specification and defined with reference to the claims below. It may in particular be appreciated that a holder, which herein has been described as comprising two members, a holder member and a locking pin, can be in the form of a single member, which then is releasably engaged in a locking member, e.g. threaded or keyed into a locking member. Similarly, an actuation assembly, which herein also has been described as comprising two members, a retractor and a holder, can be in the form of a single member, which then is releasably engaged in a proximal portion of a tubular member, e.g. threaded or keyed into a tubular member. It may also be pointed out that the mechanical insertion and delivery assembly or tool described herein could, with minor modifications, be used to operate a closure device that is of a unitary construction, i.e. with an integrated locking member. In fact, the locking member described above could be regarded as part of a holder to releasably engage the holder in a distal portion of a tubular member. An insertion assembly comprising a spring that effectuates a relative movement between two members could also be used in combination with other types of closure devices than the closure device described herein, and in particular in combination with a closure device that comprises an expandable distal portion and an expandable proximal portion, which are designed to be positioned on each side of a tissue wall. As has been explained above, it is especially advantageous that the spring force of the spring is adapted to the closure device such that the spring can accomplish a closed, reversible configuration of the closure device, but cannot accomplish a locked, non-reversible configuration of said closure device. Other parts, in particular a locking pin and a holder member, of an insertion assembly could also be used in combination with other types of closure devices, and in particular closure devices comprising an expandable distal portion and an expandable proximal portion.

## Claims

1. Medical apparatus (100) comprising:
a closure device (20; 102) having a longitudinal central axis and comprising a tubular member (21; 121) having a length and an expandable distal portion extending between a distal end portion (24; 124) and a central portion (25; 125) and an expandable proximal portion extending between said central portion and a proximal end portion (26; 126),
an insertion assembly (101) comprising a holder (106) and an actuator (107), the holder being releasably engaged in the distal end portion and the actuator being releasably engaged with the proximal end portion,
and, in response to a relative movement between the holder and the actuator, the closure device is reversibly movable between a first elongated introduction configuration and a second positioning configuration in which the distal and proximal end portions have been moved towards each other such that said expandable distal and proximal portions have expanded radially away from said longitudinal central axis, **characterized in that**
the insertion assembly further comprises a spring (105) which effectuates a relative movement between the holder and the actuator, where said relative movement causes the closure device to move from the first elongated introduction configuration to the second positioning configuration, where the spring is maximally compressed in the first configuration and the transition to the second configuration is accomplished during expansion of the spring, where said expandable distal and proximal portions have expanded radially away from said longitudinal central axis.

2. A medical apparatus according to claim 1, **characterized in that** the closure device further comprises a locking member (30), which has a distal end (32) and a proximal end (34), and said distal and proximal ends comprise radial protrusions, and said distal end (32) has at least one outer cross-sectional dimension larger than at least one inner cross-sectional dimension of the distal end portion (24) and said proximal end (34) has at least one outer cross-sectional dimension larger than at least one inner cross-sectional dimension of the proximal end portion (26), and the distance between the first and second ends is smaller than the length of the tubular member.

3. A medical apparatus according to claim 2, **characterized in that** the closure device has a third closed configuration in which the locking member is positioned in the tubular member such that the distal end portion (24) abuts the radial protrusions of the distal end (32) and at least one enlarged inner cross-sectional dimension of the proximal end (34) prevents further compression of the tubular member.

4. A medical apparatus according to claim 3, **characterized in that** the medical closure device has a final locked configuration in which one end portion of the tubular member has been moved over the radial protrusions of one end of the locking member such that the closure device is held in an expanded and locked configuration.

5. A medical apparatus according to claim 4, **characterized in that** a spring force of the spring is at least that which is required to move the closure device from the initial elongated configuration to the closed configuration, but below that which is required to place the closure device in the locked configuration.

6. A medical apparatus according to claim 1, **characterized in that** the holder comprises a locking pin (141) and a tubular holder member (143) having a distal end provided with grip members (149), and the locking pin is adapted to be disposed inside the tubular holder member, to prevent the grip members from approaching each other.

7. A medical apparatus according to claim 2, **characterized in that** the actuator comprises a pusher (146) and a retractor (145), the distal end of which is upended, and wherein the proximal end portion of the tubular member is adapted to be fixated between the upended distal end of the retractor and the distal end of the pusher.

8. A medical apparatus according to claim 7, **characterized in that** the retractor is slidably arranged inside the pusher, such that, when the pusher is moved closer to the distal end of the holder and the retractor abuts the radial protrusions of the proximal end of the locking member, the pusher pushes the proximal end portion of the tubular member over the radial protrusions of the proximal end of the locking member.

9. A medical apparatus according to claim 1, **characterized in that** the closure device is adapted for closing a septal defect in a heart.

10. A medical apparatus according to claim 1, **characterized in that** the closure device is adapted for closing a puncture in a vessel wall.

## Patentansprüche

1. Medizinisches Gerät (100), umfassend:
eine Verschlussvorrichtung (20; 102) mit einer Längsmittelachse und einem röhrenförmigen Element (21; 121) mit einer Länge und einem expandierbaren distalen Abschnitt, der sich zwischen einem distalen Endabschnitt (24; 124) und einem mittleren Abschnitt (25; 125) erstreckt, und mit einem expandierbaren proximalen Abschnitt, der sich zwischen dem mittleren Abschnitt und dem proximalen Endabschnitt (26; 126) erstreckt,
eine Einführanordnung (101) mit einem Halter (106) und einem Betätigungselement (107), wobei der Halter lösbar in den distalen Endabschnitt eingreift und das Betätigungselement lösbar mit dem proximalen Endabschnitt in Eingriff steht,
und wobei die Verschlussvorrichtung als Reaktion auf eine Relativbewegung zwischen dem Halter und dem Betätigungselement reversibel zwischen einer ersten länglichen Einführkonfiguration und einer zweiten Positionierungskonfiguration bewegbar ist, in der die distalen und proximalen Endabschnitte so zueinander hin bewegt wurden, dass die expandierbaren distalen und proximalen Abschnitte sich radial von der Längsmittelachse weg aufgeweitet haben, **dadurch gekennzeichnet, dass** die Einführanordnung ferner eine Feder (105) umfasst, die eine Relativbewegung zwischen dem Halter und dem Betätigungselement auslöst, wobei die Relativbewegung dazu führt, dass sich die Verschlussvorrichtung von der ersten länglichen Einführkonfiguration in die zweite Positionierungskonfiguration bewegt, wobei die Feder in der ersten Konfiguration maximal komprimiert ist und der Übergang zur zweiten Konfiguration während der Expansion der Feder erfolgt, wobei die expandierbaren distalen und proximalen Abschnitte sich radial von der Längsmittelachse weg aufgeweitet haben.

2. Medizinisches Gerät nach Anspruch 1, **dadurch gekennzeichnet, dass** die Verschlussvorrichtung ferner ein Arretierungselement (30) umfasst, das ein distales Ende (32) und ein proximales Ende (34) aufweist, und dass die distalen und proximalen Enden radiale Vorsprünge umfassen, und dass das distale Ende (32) mindestens eine äußere Querschnittsausdehnung aufweist, die größer ist als mindestens eine innere Querschnittsausdehnung des distalen Endabschnitts (24), und dass das proximale Ende (34) mindestens eine äußere Querschnittsausdehnung aufweist, die größer ist als mindestens eine innere Querschnittsausdehnung des proximalen Endabschnitts (26), und dass der Abstand zwischen dem ersten und dem zweiten Ende kleiner ist als die Länge des röhrenförmigen Elements.

3. Medizinisches Gerät nach Anspruch 2, **dadurch gekennzeichnet, dass** die Verschlussvorrichtung eine dritte geschlossene Konfiguration aufweist, in der das Arretierungselement so im röhrenförmigen Element positioniert ist, dass der distale Endabschnitt (24) an den radialen Vorsprüngen des distalen Endes (32) anliegt und dass mindestens eine vergrößerte innere Querschnittsausdehnung des proximalen Endes (34) eine weitere Kompression des röhrenförmigen Elements verhindert.

4. Medizinisches Gerät nach Anspruch 3, **dadurch gekennzeichnet, dass** die medizinische Verschlussvorrichtung eine endgültige verschlossene Konfiguration aufweist, in der ein Endabschnitt des röhrenförmigen Elements über die radialen Vorsprünge eines Endes des Arretierungselements hinaus bewegt worden ist, so dass die Verschlussvorrichtung in einer expandierten und arretierten Konfiguration gehalten wird.

5. Medizinisches Gerät nach Anspruch 4, **dadurch gekennzeichnet, dass** eine Federkraft der Feder mindestens der Kraft entspricht, die benötigt wird, um die Verschlussvorrichtung aus der anfänglichen länglichen Konfiguration in die geschlossene Konfiguration zu bewegen, aber die geringer ist als die Kraft, die benötigt wird, um die Verschlussvorrichtung in die arretierte Konfiguration zu setzen.

6. Medizinisches Gerät nach Anspruch 1, **dadurch gekennzeichnet, dass** der Halter einen Arretierungsstift (141) und ein röhrenförmiges Halteelement (143) mit einem distalen Ende mit Griffelementen (149) umfasst und dass der Arretierungsstift im röhrenförmigen Halteelement angeordnet werden kann, um zu verhindern, dass sich die Griffelemente einander annähern.

7. Medizinisches Gerät nach Anspruch 2, **dadurch gekennzeichnet, dass** das Betätigungselement einen Schieber (146) und einen Retraktor (145) umfasst, dessen distales Ende umgestülpt ist, und worin der proximale Endabschnitt des röhrenförmigen Elements zwischen dem umgestülpten distalen Ende des Retraktors und dem distalen Ende des Schiebers befestigt werden kann.

8. Medizinisches Gerät nach Anspruch 7, **dadurch gekennzeichnet, dass** der Retraktor verschiebbar im Schieber angeordnet ist, so dass der Schieber den proximalen Endabschnitt des röhrenförmigen Elements über die radialen Vorsprünge des proximalen Endes des Arretierungselements schiebt, wenn der Schieber näher an das distale Ende des Halters geschoben wird und der Retraktor an den radialen Vorsprüngen des proximalen Endes des Arretierungselements anliegt.

9. Medizinisches Gerät nach Anspruch 1, **dadurch gekennzeichnet, dass** die Verschlussvorrichtung einen Septumdefekt in einem Herz verschließen kann.

10. Medizinisches Gerät nach Anspruch 1, **dadurch gekennzeichnet, dass** die Verschlussvorrichtung eine Punktion in einer Gefäßwand verschließen kann.

## Revendications

1. Appareil médical (100), comprenant :
un dispositif de fermeture (20 ; 102) ayant un axe central longitudinal et comprenant un organe tubulaire (21 ; 121) ayant une longueur et une portion distale extensible s'étendant entre une portion d'extrémité distale (24 ; 124) et une portion centrale (25 ; 125) et une portion proximale extensible s'étendant entre ladite portion centrale et une portion d'extrémité proximale (26 ; 126),
un ensemble d'insertion (101) comprenant un support (106) et un actionneur (107), le support étant engagé de manière libérable dans la portion d'extrémité distale et l'actionneur étant engagé de manière libérable avec la portion d'extrémité proximale,
et, en réponse à un mouvement relatif entre le support et l'actionneur, le dispositif de fermeture étant déplaçable de manière inversable entre une première configuration d'introduction allongée et une deuxième configuration de positionnement dans laquelle les portions d'extrémité distale et proximale ont été déplacées l'une vers l'autre de telle sorte que lesdites portions distale et proximale extensibles se soient étirées radialement à l'écart dudit axe central longitudinal, **caractérisé en ce que**
l'ensemble d'insertion comprend en outre un ressort (105) qui effectue un mouvement relatif entre le support et l'actionneur, ledit mouvement relatif causant le déplacement du dispositif de fermeture depuis la configuration d'introduction allongée dans la deuxième configuration de positionnement, le ressort étant comprimé au maximum dans la première configuration et la transition dans la deuxième configuration étant effectuée au cours de l'extension du ressort, lesdites portions distale et proximale extensibles s'étant étirées radialement à l'écart dudit axe central longitudinal.

2. Appareil médical selon la revendication 1, **caractérisé en ce que** le dispositif de fermeture comprend en outre un organe de verrouillage (30) qui a une extrémité distale (32) et une extrémité proximale (34), et lesdites extrémités distale et proximale comprennent des saillies radiales, et ladite extrémité distale (32) à au moins une dimension extérieure en section transversale qui est plus grande qu'au moins une dimension intérieure en section transversale de la portion d'extrémité distale (24) et ladite extrémité proximale (34) a au moins une dimension extérieure en section transversale qui est plus grande qu'au moins une dimension intérieure en section transversale de la portion d'extrémité proximale (26), et la distance entre les première et deuxième extrémités est plus petite que la longueur de l'organe tubulaire.

3. Appareil médical selon la revendication 2, **caractérisé en ce que** le dispositif de fermeture a une troisième configuration fermée dans laquelle l'organe de verrouillage est positionné dans l'organe tubulaire de telle sorte que la portion d'extrémité distale (24) vienne buter contre les saillies radiales de l'extrémité distale (32) et au moins une dimension intérieure agrandie en section transversale de l'extrémité proximale (34) empêche une compression plus poussée de l'organe tubulaire.

4. Appareil médical selon la revendication 3, **caractérisé en ce que** le dispositif de fermeture médical a une configuration verrouillée finale dans laquelle une portion d'extrémité de l'organe tubulaire a été déplacée par-dessus les saillies radiales d'une extrémité de l'organe de verrouillage de telle sorte que le dispositif de fermeture soit maintenu dans une configuration étirée et verrouillée.

5. Appareil médical selon la revendication 4, **caractérisé en ce qu'**une force de ressort du ressort est au moins égale à la force requise pour déplacer le dispositif de fermeture de sa configuration allongée initiale dans la configuration fermée, mais est inférieure à la force requise pour placer le dispositif de fermeture dans la configuration verrouillée.

6. Appareil médical selon la revendication 1, **caractérisé en ce que** le support comprend une goupille de verrouillage (141) et un organe de support tubulaire (143) ayant une extrémité distale pourvue d'organes de préhension (149), et la goupille de verrouillage est prévue pour être disposée à l'intérieur de l'organe de support tubulaire, pour empêcher les organes de préhension de se rapprocher l'un de l'autre.

7. Appareil médical selon la revendication 2, **caractérisé en ce que** l'actionneur comprend un poussoir (146) et un rétracteur (145) dont l'extrémité distale est redressée, et la portion d'extrémité proximale de l'organe tubulaire étant prévue pour être fixée entre l'extrémité distale redressée du rétracteur et l'extrémité distale du poussoir.

8. Appareil médical selon la revendication 7, **caractérisé en ce que** le rétracteur est agencé à coulissement à l'intérieur du poussoir de telle sorte que lorsque le poussoir est amené plus près de l'extrémité distale du support et que le rétracteur vient buter contre les saillies radiales de l'extrémité proximale de l'organe de verrouillage, le poussoir pousse la portion d'extrémité proximale de l'organe tubulaire par-dessus les saillies radiales de l'extrémité proximale de l'organe de verrouillage.

9. Appareil médical selon la revendication 1, **caractérisé en ce que** le dispositif de fermeture est prévu pour fermer un défaut septal dans le cour.

10. Appareil médical selon la revendication 1, **caractérisé en ce que** le dispositif de fermeture est prévu pour fermer une perforation dans une paroi d'un vaisseau.
